(19) Europäisches Patentamt
European Patent Office
Office européen des brevets

(11) Numéro de publication : **0 395 529 B1**

# (12) FASCICULE DE BREVET EUROPEEN

(45) Date de publication du fascicule du brevet : **03.11.93 Bulletin 93/44**

(51) Int. Cl.$^5$ : **C07C 217/60,** A61K 31/135

(21) Numéro de dépôt : **90401153.3**

(22) Date de dépôt : **27.04.90**

**(54) Nouveau dérivé d'hydroxyphénéthylamine et ses sels, procédé de préparation, application à titre de médicaments et utilisation comme outil pharmacologique spécifique.**

(30) Priorité : **28.04.89 FR 8905649**

(43) Date de publication de la demande : **31.10.90 Bulletin 90/44**

(45) Mention de la délivrance du brevet : **03.11.93 Bulletin 93/44**

(84) Etats contractants désignés : **AT BE CH DE DK ES GB GR IT LI LU NL SE**

(56) Documents cités :
**EP-A- 0 258 096**
**FR-A- 2 356 417**
**FR-A- 2 436 773**

(73) Titulaire : **ROUSSEL-UCLAF**
**35, Boulevard des Invalides**
**F-75007 Paris (FR)**

(72) Inventeur : **Cosquer, Philippe**
**Cité Jacques Duclos, Esc.8, Porte 624**
**F-93200 Saint Denis (FR)**
Inventeur : **Delevallée, Françoise**
**48,50, Avenue de la Dame Blanche**
**F-94120 Fontenay sous Bois (FR)**
Inventeur : **Droux, Serge**
**8, Rue du Docteur Jean Perlés**
**F-93600 Aulnay sous Bois (FR)**
Inventeur : **Fortin, Michel**
**12, Passage Cottin**
**F-75018 Paris (FR)**
Inventeur : **Petit, Francis**
**119, Avenue Carnot**
**F-93140 Bondy (FR)**

(74) Mandataire : **Bourgouin, André et al**
**Département des Brevets ROUSSEL UCLAF**
**111, route de Noisy B.P. no 9**
**F-93230 Romainville (FR)**

EP 0 395 529 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un nouveau dérivé d'hydroxyphénéthylamine et ses sels, leur procédé de préparation, leur application à titre de médicaments et leur utilisation comme outil pharmacologique spécifique.

L'invention a pour objet un nouveau dérivé d'hydroxyphénétylamine caractérisé en ce qu'il répond à la formule (I) :

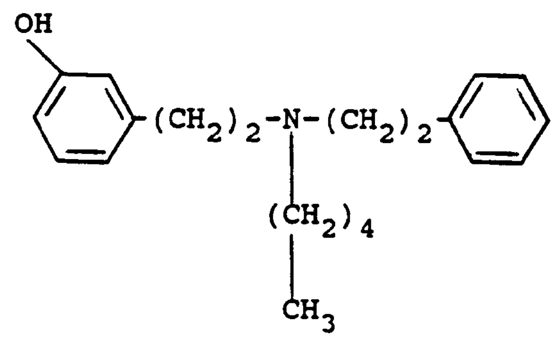

(I)

ainsi que ses sels d'addition avec les acides minéraux ou organiques.

Les sels d'addition avec les acides minéraux ou organiques peuvent être, par exemple, les sels formés avec les acides chlorhydrique, bromhydrique, iodhydrique, nitrique, sulfurique, phosphorique, acétique, formique, benzoïque, maléique, fumarique, succinique, tartrique, citrique, oxalique, glyoxylique, aspartique, alcanesulfoniques, tels que l'acide méthane sulfonique et arylsulfonique, tels que l'acide benzène sulfonique.

La présente invention a notamment pour objet :

- le N-pentyl N-phénéthyl 3-hydroxy phénéthylamine ainsi que :
- le chlorhydrate de N-pentyl N-phénéthyl 3-hydroxy phénéthylamine.

L'invention a également pour objet un procédé de préparation du produit de formule (I) telle que définie à la revendication 1, ainsi que de ses sels d'addition avec les acides minéraux et organiques, caractérisé en ce que l'on déméthyle le produit de formule (II) :

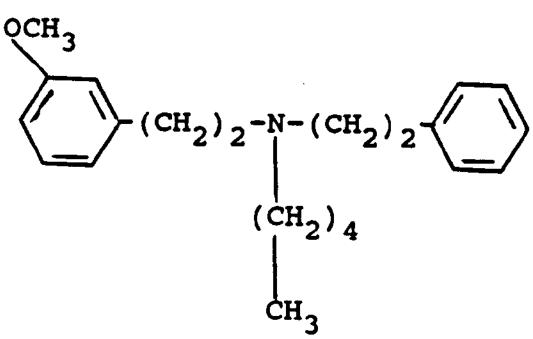

(II)

pour obtenir le produit de formule (I) que l'on peut isoler sous forme de base et, si désiré, salifier.

Dans les conditions préférentielles de mise en oeuvre de l'invention, le procédé de préparation ci-dessus décrit est caractérisé en ce que la déméthylation du produit de formule (II) est effectuée au moyen d'une solution concentrée d'acide bromhydrique, au reflux du mélange réactionnel.

Le produit de formule (I) présente un caractère basique. On peut avantageusement préparer les sels d'addition de ce produit, en faisant réagir en proportions sensiblement stoechiométriques, un acide minéral ou organique avec ledit produit.

Le produit de formule (I), ainsi que ses sels, objet de la présente invention, possède de très intéressantes propriétés pharmacologiques : il présente en particulier une affinité antagoniste pour le récepteur opiacé kappa remarquablement dissociée.

Le produit de formule (I), ainsi que ses sels, objet de la présente invention, présente une faible affinité pour le récepteur opiacé mu et pratiquement pas pour le récepteur dopaminergique $D_2$.

Ces propriétés sont illustrées plus loin dans la partie expérimentale.

Ces remarquables propriétés justifient l'utilisation de ce produit, objet de la présente demande, et de ses

sels, à titre de médicaments.

La présente demande a ainsi pour objet l'application à titre de médicaments, de ce nouveau dérivé d'hydroxyphénéthylamine tel que défini par la formule (I), ainsi que des sels d'addition avec les acides pharmaceutiquement acceptables de ce dérivé.

Les médicaments, objet de la présente invention, trouvent par exemple leur emploi dans le cas de traitement intensif avec un produit agoniste du récepteur opiacé kappa, ce traitement nécessitant une interruption pour des raisons, par exemple, de surdosage.

Un tel type de médicament agoniste kappa est décrit par exemple dans le brevet français n° 2 603 035 et est utilisé notamment pour combattre une douleur d'origines diverses, par exemple une douleur de nature musculaire, articulaire ou nerveuse. Un autre exemple d'agoniste kappa est le produit : U - 50 488 qui possède en plus de la propriété analgésique une propriété diurétique.

Le U - 50488 est le trans-3,4-dichloro-N-méthyl-N-[2-(1-pyrrolidinyl) cyclohexyl]-benzène acétamide décrit par exemple dans la publication Pharmacologist 23(3) : 112 Abs, 1981.

L'effet du produit antagoniste kappa, objet de la présente invention, est alors de déplacer spécifiquement le produit agoniste par compétition au niveau du récepteur kappa, jouant ainsi le rôle d'un antidote.

Ainsi, le produit et ses sels, objets de l'invention, peuvent, par exemple, exercer un effet antagoniste vis-à-vis de l'activité analgésique ou de l'activité diurétique d'un agoniste kappa.

La dose usuelle, variable selon le produit utilisé, le sujet traité et l'affection en cause peut être, par exemple de 5 mg à 200 mg par jour, par voie orale ou parentérale chez l'homme.

L'invention a également pour objet les compositions pharmaceutiques qui renferment le dérivé précité ou l'un de ses sels d'addition avec les acides pharmaceutiquement acceptables, à titre de principe actif.

A titre de médicaments, le dérivé répondant à la formule (I) et ses sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les capsules, les granulés, les suppositoires, les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

De plus, les produits, objets de l'invention, en raison de leur reconnaissance spécifique du récepteur opiacé kappa, peuvent être utilisés comme outil pharmacologique spécifique. Cette propriété les rend tout particulièrement précieux pour les expériences en laboratoire qui nécessitent une haute spécificité du produit de référence utilisé, ce que ne possèdent pas les produits de référence les plus connus comme antagonistes du récepteur opiacé kappa tels que la NALOXONE ou MR2266 qui reconnaissent, en particulier, le récepteur opiacé mu.

La NALOXONE est la 4,5-époxy-3,14-dihydroxy-17-(2-propényl) morphinan-6-one décrite par exemple dans le Merck Index 11th edition, monographie N° 6277.

Le MR 2266 est le chlorhydrate de (-) 5,9α-diéthyl-2-(3-furylméthyl)-2'-hydroxy-6,7-benzomorphane décrit par exemple dans Europ. J. Pharmacol 46 : 393, 15 Déc. 1977.

Ainsi l'invention a également pour objet l'utilisation du produit de formule (I) telle que définie ci-dessus comme moyen d'étude, de dosage et de localisation des récepteurs opiacés kappa.

L'invention a tout particulièrement pour objet l'utilisation du produit de formule (I) telle que définie précédemment et de ses sels d'addition avec les acides pharmaceutiquement acceptables pour la préparation de compositions pharmaceutiques destinées au traitement du surdosage d'un produit agoniste du récepteur opiacé kappa.

L'invention a enfin pour objet à titre de produit industriel nouveau, le produit de formule (II) :

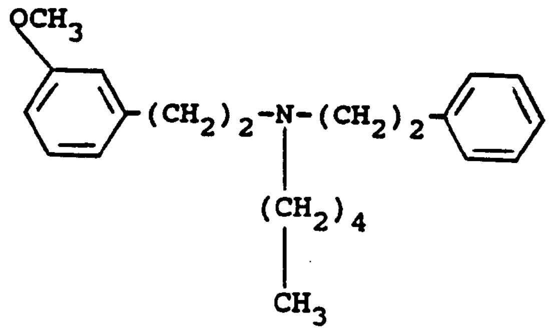

(II)

utile notamment pour la préparation du produit de formule (I).

Le produit de formule (II) peut être préparé par un procédé caractérisé en ce que l'on fait réagir le produit de formule (III) :

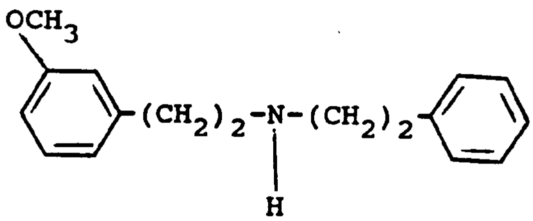

(III)

avec un halogénure de formule (IV) :

$$Hal\text{-}(CH_2)_4\text{-}CH_3 \qquad (IV)$$

dans laquelle Hal représente un atome de chlore, de brome ou d'iode pour obtenir le produit de formule (II) recherché.

La préparation du produit de formule (III) est décrite dans le brevet français n° 2 356 417.

Il va être donné, à titre non limitatif, des exemples de mise en oeuvre de l'invention.

**Exemple 1 : N-pentyl N-phénéthyl 3-hydroxy phénéthylamine**

Stade A : préparation du bromhydrate de N-phénéthyl 3-méthoxy phénéthylamine.

Un exemple de cette préparation est donné au stade A de l'exemple 1 du brevet français n° 2 356 417.

Stade B : préparation du N-pentyl N-phénéthyl 3-méthoxy phénéthylamine.

Une quantité de 13,4 g de bromhydrate de N-phénéthyl 3-méthoxy phénéthylamine est portée au reflux dans 200 $cm^3$ d'acétone en présence de 22 $cm^3$ d'iodure de pentyle et de 29,3 g de carbonate de potassium pendant 4 heures. Le milieu est filtré, lavé à l'acétone et le filtrat concentré à sec sous pression réduite. La quantité de 30 g de produit brut ainsi obtenue est chromatographiée sur silice (éluant : cyclohexane-acétate d'éthyle 7-3) et la fraction ainsi isolée du produit pur recherché soit le N-pentyl N-phénéthyl 3-méthoxy phénéthylamine est de 11,4 g.

Stade C : préparation du N-pentyl N-phénéthyl 3-hydroxy phénéthylamine

Une quantité de 6,7 g de N-pentyl N-phénéthyl 3-méthoxy phénéthylamine est portée au reflux dans 40 $cm^3$ d'une solution aqueuse d'acide bromhydrique à 48% pendant une heure et demie à la température de +150°C. Après refroidissement du milieu à température ambiante, l'extraction est faite au chlorure de méthylène et les liqueurs chlorométhyléniques obtenues sont séchées sur sulfate de sodium , filtrées puis concentrées à sec sous pression réduite.

Les 8,55 g de produit brut obtenus sont purifiés par chromatographie sur silice (éluant : chlorure de méthylène-tétrahydrofuranne 1-1) pour donner 3,2 g de produit que l'on dissout à chaud dans 3 $cm^3$ d'isopropanol puis recristallise à la température ambiante.

Après essorage suivi de lavage par deux fois avec 3 $cm^3$ d'isopropanol glacé puis séchage à +50°C sous pression réduite, la fraction ainsi isolée du produit recherché soit le N-pentyl N-phénéthyl 3-hydroxy phénéthylamine est de 1,72g.
F = 82-84°C.

| | | | | |
|---|---|---|---|---|
| Analyse : | $C_{21}H_{29}NO$ | PM = 311,471 | | |
| Calculé : | C% 80,98 | H% 9,38 | N% 4,49 | |
| Trouvé : | 81,20 | 9,60 | 4,40 | |

Exemple 2 : **chlorhydrate de N-pentyl N-phénéthyl 3-hydroxy phénéthylamine**

On place une quantité de 15 g de N-pentyl N-phénéthyl 3-hydroxy phénéthylamine décrite à l'exemple 1 dans 45 $cm^3$ d'eau et ajoute 4 $cm^3$ d'acide chlorhydrique concentré. La suspension est laissée une nuit à température ambiante sous agitation.

Le produit obtenu est ensuite glacé une heure à une température comprise entre 0° et +5°C puis essoré et rincé par 35 $cm^3$ d'eau glacée à environ +5°C. Après séchage, on obtient 16,29 g de chlorhydrate de N-pentyl N-phénéthyl 3-hydroxy phénéthylamine. F = 115°C.

| | | | | |
|---|---|---|---|---|
| Analyse : | $C_{21}H_{30}NOCl$ | PM = 347,93 | | |
| Calculé : | C% 72,5 | H% 8,69 | N% 4,03 | Cl% 10,19 |
| Trouvé : | 72,7 | 8,8 | 3,9 | 10,3 |

**EXEMPLE 3 :**

On a préparé des comprimés répondant à la formule suivante :
- Produit de l'exemple 1     100 mg
- Excipient q.s.p.     400 mg.

(Detail de l'excipient : lactose, talc, amidon, stéarate de mégnasium).

**EXEMPLE 4 :**

On a préparé un soluté injectable (voie intra-musculaire) répondant à la formule suivante :
- Produit de l'exemple 1     50 mg
- Solvant stérile q.s.p.     5 $cm^3$.

**Etude pharmacologique**

1) Effet antagoniste vis-à-vis de l'activité diurétique de U-50 488

Action sur la diurèse

L'essai est réalisé sur des rats mâles de poids corporel moyen de 150 g à jeûn depuis 16 heures mais ayant de l'eau ad libitum comme boisson.

Le produit de l'exemple 1 à 20 mg/kg SC et le U-50488 à 5 mg/kg PO sont administrés simultanément à un groupe de 12 rats, un deuxième groupe d'animaux est traité uniquement par le U-50488 à 5 mg/kg PO, tandis qu'un autre lot reçoit seulement une injection sous-cutanée de produit de l'exemple 1 à 20 mg/kg. Un lot de rats témoins reçoit les véhicules correspondant aux deux produits. Les rats sont ensuite placés dans des cages à diurèse par groupe de deux et le volume urinaire est recueilli pendant une heure.

| | Dose mg/kg SC | Volume urinaire excrété en 1 heure (ml/kg) m ± esm |
|---|---|---|
| Témoins | 0 | 6,5 ± 0,2 |
| Produit de l'exemple 1 | 20 | 6,7 ± 0,2 |
| U-50 488 | 5 | 9,8 ± 0,3 |
| U-50 488 + produit de l'exemple 1 | 5 + 20 | 5,7 ± 0,2 |

2) Effet antagoniste vis-à-vis de l'activité analgésique de U-50 488

Test de la plaque chaude chez la souris

Des souris femelles sont placées une par une sur une plaque de cuivre maintenue à 56°C à l'aide d'un bain-marie thermostaté. La réaction à la douleur se manifeste par le léchage d'une ou des deux pattes antérieures. Le temps de réaction au stimulus thermique et noté et l'on ne retient que les animaux réagissant entre 4,5 et 6,5 secondes. Les animaux sont ensuite répartis par groupes homogènes. Un groupe d'animaux témoins reçoit le U-50 488 à 5 mg/kg SC, un autre groupe le produit de l'exemple 1 à 20 mg/kg SC, tandis que les deux produits sont administrés simultanément par voie SC à un troisième lot d'animaux à ces mêmes doses. Un groupe d'animaux témoins reçoit les injections de véhicule correspondant.

Le temps de réaction à la douleur est mesuré trente minutes après injection des produits. Les variations du temps de réaction sont exprimées en pourcentage du temps initial, compte tenu des variations du groupe témoin.

| | Dose mg/kg SC | Temps de réaction (s) m ± esm 0 mn | 30 mn |
|---|---|---|---|
| Témoins | 0 | 5,2 ± 0,1 | 5,0 ± 0,2 |
| Produit de l'exemple 1 | 20 | 5,1 ± 0,1 | 4,7 ± 0,6 |
| U-50 488 | 5 | 5,2 ± 0,1 | 9,6 ± 1,1 |
| U-50 488 + produit de l'exemple 1 | 5 + 20 | 5,1 ± 0,2 | 6,7 ± 0,6 * |

* $p < 0,05$ selon le test de Student par rapport au groupe ayant été traité par le U-50 488.

3) Mise en évidence de la propriété de dissociation des activités Kappa, μ et dopaminergiques soit de la seule affinité pour le récepteur opiacé Kappa :

a) Test de liaison au récepteur opiacé Kappa in vitro

On utilise des culots membranaires conservés à -30°C (éventuellement pendant environ 30 jours) et préparés à partir de cervelets de cobayes.

Ces culots sont remis en suspension dans le tampon Tris pH 7,7. On répartit les fractions de 2 $cm^3$ dans des tubes à hémolyse et ajoute de la $^3H$ éthylkétocyclazocine 1 nM et le produit à étudier. (Le produit est d'abord testé à $5 \times 10^{-6}$M en triple). Lorsque le produit testé déplace de plus de 50 % la radioactivité liée spécifiquement au récepteur, il est testé à nouveau selon une gamme de 7 doses afin de déterminer la dose qui inhibe de 50 % la radioactivité liée spécifiquement au récepteur. On détermine ainsi la concentration inhibitrice 50 %).

La liaison non spécifique est déterminée par addition de produit connu sous le nom U-50 488 H (Lahti et al. 1982, Life Sci. 31, 2257) à $10^{-5}$M (en triple). On incube à 25°C pendant 40 minutes, remet au bain-marie à

0°C, 5 minutes, filtre sous vide, rince au tampon Tris pH 7,7 et compte la radioactivité en présence du scintillant Triton.

Le résultat est exprimé directement en concentration inhibitrice 50 % ($CI_{50}$), c'est-à-dire en concentration de produit étudié, exprimée en nM, nécessaire pour déplacer 50 % de la radioactivité spécifique fixée sur le récepteur étudié.

Résultat :

La $CI_{50}$ trouvée pour le produit de l'exemple 1 est de 57 nanomoles.

b) Test de liaison au récepteur opiacé mu in vitro

On utilise des culots membranaires conservés à -30°C (éventuellement pendant environ 30 jours) et préparés à partir de cerveaux de rats.

Ces culots sont remis en suspension dans le tampon Tris pH 7,7. On répartit des fractions de 2 $cm^3$ dans des tubes à hémolyse et ajoute de la $^3H$ DAGO (1 nM) et le produit à étudier. (Le produit est d'abord testé à 5 x $10^{-6}$M, en triple). Lorsque le produit testé déplace de plus de 50 % la radioactivite liée spécifiquement au récepteur, il est testé à nouveau selon une gamme de 7 doses afin de déterminer la dose qui inhibe de 50 % la radioactivité liée spécifiquement au récepteur. On détermine ainsi la concentration inhibitrice 50 %).

La liaison non spécifique est déterminée par addition de morphine (CHILDERS S.R. Eur. J. Pharmacol. 55, 11) à $10^{-5}$M (en triple). On incube à 25°C pendant 40 minutes, remet au bain-marie à 0°C, 5 minutes, filtre sous vide, rince au tampon Tris pH 7,7 et compte la radioactivité en présence du scintillant Triton.

Le résultat est exprimé directement en concentration inhibitrice 50 % ($CI_{50}$), c'est-à-dire en concentration de produit étudié, exprimée en nM, nécessaire pour déplacer 50 % de la radioactivité spécifique fixée sur le récepteur étudié.

Résultat :

La $CI_{50}$ trouvée pour le produit de l'exemple 1 est de 840 nanomoles.

c) Test de liaison au récepteur à la dopamine in vitro

On utilise des culots membranaires conservés à -30°C (éventuellement pendant environ 30 jours) et préparés à partir de corps striés de rats.

Ces culots sont remis en suspension dans le tampon Krebs Tris pH 7,25. Après une préincubation de 10 minutes à 37°C, on répartit en des fractions de 2 $cm^3$ dans des tubes à hémolyse et ajoute des $^3H$ spiropéridol (0,15 nM) et le produit à étudier. (Le produit est d'abord testé à 5 x $10^{-6}$M, en triple). Lorsque le produit testé déplace de plus de 50 % la radioactivité liée spécifiquement au récepteur, il est testé à nouveau selon une gamme de 7 doses afin de déterminer la dose qui inhibe de 50 % la radioactivité liée spécifiquement au récepteur. On détermine ainsi la concentration inhibitrice 50 %).

La liaison non spécifique est déterminée par addition de halopéridol (SIELDS J.Z. and Co BRAIN RESEARCH 1977, 136, p. 578-584) à $10^{-5}$M (en triple). On incube à 27°C pendant 20 minutes, remet au bain-marie à 0°C, 5 minutes, filtre sous vide, rince au tampon Tris pH 7,7 et compte la radioactivité en présence du scintillant Triton.

Le résultat est exprimé directement en concentration inhibitrice 50 % ($CI_{50}$), c'est-à-dire en concentration de produit étudié, exprimée en nM, nécessaire pour déplacer 50 % de la radioactivité spécifique fixée sur le récepteur étudié.

Résultat :

La $CI_{50}$ trouvée pour le produit de l'exemple 1 est de 10600 nanomoles.

Conclusion :

Le produit de l'exemple 1 n'est affine que pour le récepteur opiacé Kappa.

4) Test de distinction entre agoniste et antagoniste au récepteur opiacé Kappa

L'utilisation d'un ligand antagoniste permet de distinguer in vitro si un produit est agoniste ou antagoniste ; dans ce cas en effet un agoniste verra sa $CI_{50}$ augmenter dans un rapport important (15 à 50) selon qu'il est incubé en absence ou en présence de NaCl et/ou GTP, alors que la $CI_{50}$ d'un antagoniste variera peu dans les 2 conditions.

On utilise des culots membranaires conservés à -30°C (éventuellement pendant environ 30 jours) et préparés à partir de cervelets de cobayes, avec ou sans NaCl 100 mM, GTP 50 uM.

Ces culots sont remis en suspension dans le tampon Tris 50 mM pH 7,7. On répartit des fractions de 2 $cm^3$ dans des tybes à hémolyse et ajoute de la $^3H$ diprénorphine (0,3 nM) et le produit à étudier. (Le produit est d'abord testé à 5 x $10^{-6}$M, en triple). Lorsque le produit testé déplace de plus de 50 % la radioactivité liée spécifiquement au récepteur, il est testé à nouveau selon une gamme de 7 doses afin de déterminer la dose qui inhibe de 50 % la radioactivité liée spécifiquement au récepteur. On détermine ainsi la concentration inhibitrice 50 %).

La liaison non spécifique est déterminée par addition de produit appelé U-50 488 (FRANCES B.C. et al. Eur. J. Pharmacol. 1985, 117, 223) à $10^{-5}$M en triple). On incube à 25°C pendant 40 minutes, remet au bain-marie à 0°C, 5 minutes, filtre sous vide, rince au tampon Tris pH 7,7 et compte la radioactivité en présence du scintillant Triton.

Le résultat est exprimé directement en concentration inhibitrice 50 % ($CI_{50}$), c'est-à-dire en concentration de produit étudié, exprimée en nM, nécessaire pour déplacer 50 % de la radioactivité spécifique fixée sur le récepteur étudié.

Résultat :

La $CI_{50}$ est de 43 nM en absence de GTP et NaCl, et de 135 nM en présence de GTP et NaCl.

Ainsi le résultat obtenu montre bien que le produit de l'exemple 1 est un produit antagoniste du récepteur Kappa.

## Revendications

**Revendications pour les Etats contractants suivants : AT, BE, CH, DE, DK, GB, IT, LI, LU, NL, SE**

**1.** Nouveau dérivé d'hydroxyphénétylamine caractérisé en ce qu'il répond à la formule (I) :

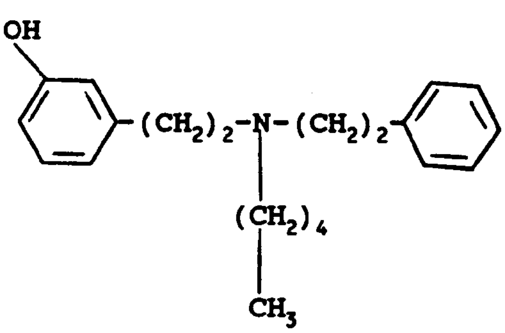

(I)

ainsi que ses sels d'addition avec les acides minéraux ou organiques.

**2.**
- le N-pentyl N-phénéthyl 3-hydroxy phénéthylamine
- le chlorhydrate de N-pentyl N-phénéthyl 3-hydroxy phénéthylamine.

**3.** Procédé de préparation du produit de formule (I) telle que définie à la revendication 1, ainsi que de ses sels d'addition avec les acides minéraux ou organiques, caractérisé en ce que l'on déméthyle le produit de formule (II) :

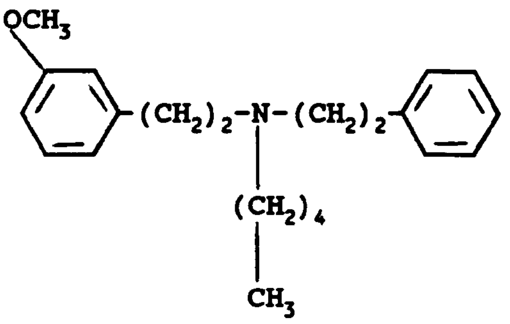

(II)

pour obtenir le produit de formule (I) que l'on peut isoler sous forme de base et, si désiré, salifier.

4. Médicaments caractérisés en ce qu'ils sont constitués par le produit de formule (I) telle que définie à la revendication 1 ainsi que par ses sels d'addition avec les acides pharmaceutiquement acceptables.

5. Compositions pharmaceutiques caractérisées en ce qu'elles renferment, à titre de principe actif, le produit de formule (I) telle que définie à la revendication 1 ou l'un de ses sels d'addition avec les acides pharmaceutiquement acceptables.

6. Utilisation du produit de formule (I) telle que définie à la revendication 1 comme moyen d'étude, de dosage et de localisation des récepteurs opiacés kappa.

7. Utilisation du produit de formule (I) telle que définie à la revendication 1 et de ses sels d'addition avec les acides pharmaceutiquement acceptables pour la préparation de compositions pharmaceutiques destinées au traitement du surdosage d'un produit agoniste du récepteur opiacé kappa.

8. A titre de produit industriel nouveau, le produit de formule (II) telle que définie à la revendication 3.

**Revendications pour l'Etat contractant suivant : ES**

1. Procédé pour préparer le dérivé répondant à la formule (I) :

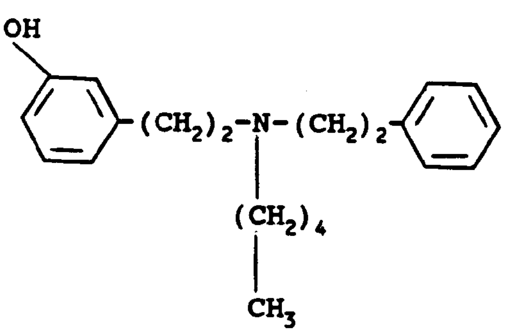

(I)

ainsi que ses sels d'addition avec les acides minéraux ou organiques, caractérisé en ce que l'on déméthyle le produit de formule (II) :

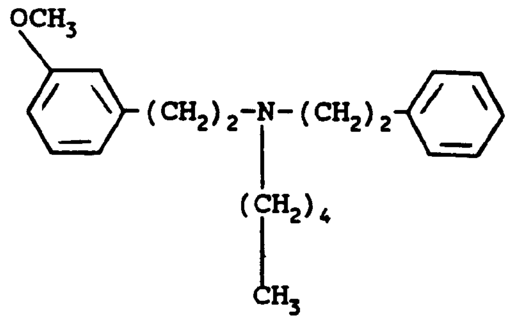

(II)

pour obtenir le produit de formule (I) que l'on peut isoler sous forme de base et, si désiré, salifier.

2. Procédé selon la revendication 1, caractérisé en ce que l'on salifie la base à l'aide d'acide chlorhydrique.

3. Utilisation du produit de formule (I) telle que définie à la revendication 1 et de ses sels d'addition avec les acides pharmaceutiquement acceptables pour la préparation de compositions pharmaceutiques destinées au traitement du surdosage d'un produit agoniste du récepteur opiacé kappa.

**Revendications pour l'Etat contractant suivant : GR**

1. Procédé pour préparer le dérivé répondant à la formule (I) :

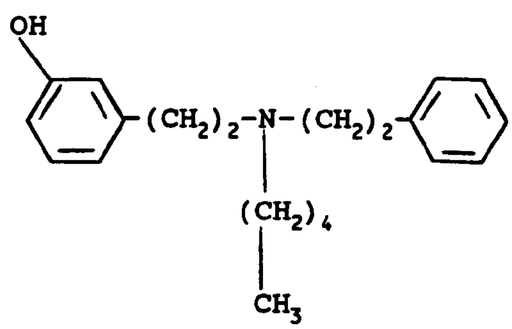

(I)

ainsi que ses sels d'addition avec les acides minéraux ou organiques, caractérisé en ce que l'on déméthyle le produit de formule (II) :

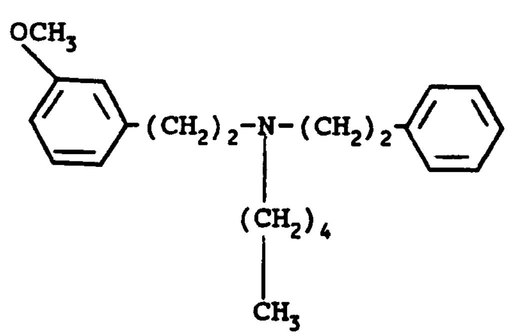

(II)

pour obtenir le produit de formule (I) que l'on peut isoler sous forme de base et, si désiré, salifier.

2. Procédé selon la revendication 1, caractérisé en ce que l'on salifie la base à l'aide d'acide chlorhydrique.

3. A titre de produit industriel nouveau, le produit de formule (II) telle que définie à la revendication 1.

4. Procédé de préparation de compositions pharmaceutiques caractérisé en ce que l'on met à titre de principe actif le produit de formule (I) ou l'un de ses sels d'addition avec les acides minéraux ou organiques pharmaceutiquement acceptables sous une forme destinée à cet usage.

5. Utilisation du produit de formule (I) telle que définie à la revendication 1 et de ses sels d'addition avec les acides pharmaceutiquement acceptables pour la préparation de compositions pharmaceutiques destinées au traitement du surdosage d'un produit agoniste du récepteur opiacé kappa.

**Patentansprüche**

**Patentansprüche für folgende Vertragsstaaten : AT, BE, CH, DE, DK, GB, IT, LI, LU, NL, SE**

1. Neues Hydroxyphenethylamin-Derivat, dadurch gekennzeichnet, daß es der Formel (I)

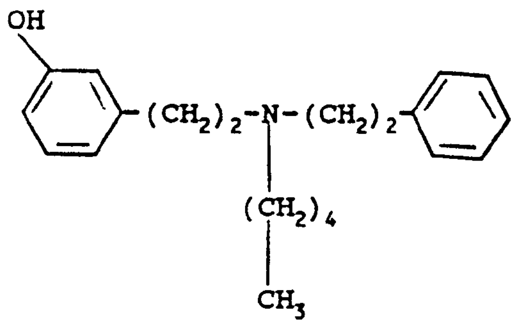

(I)

entspricht, sowie dessen Additionssalze mit Mineral- oder organischen Säuren.

2. N-Pentyl-N-phenethyl-3-hydroxyphenethylamin, N-Pentyl-N-phenethyl-3-hydroxyphenethylamin-hydrochlorid.

3. Verfahren zur Herstellung des Produkts der Formel (I), wie in Anspruch 1 definiert, sowie von dessen Additionssalzen mit Mineral- oder organischen Säuren, dadurch gekennzeichnet, daß man das Produkt der Formel (II)

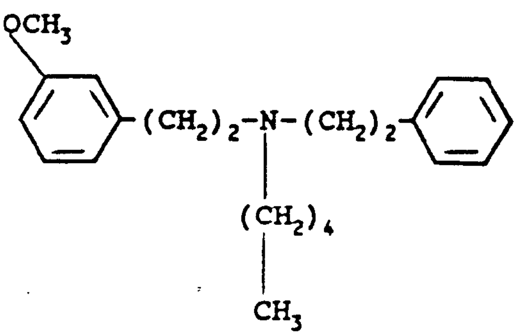

(II)

entmethyliert, um zu dem Produkt der Formel (I) zu gelangen, das man in Form der Base isolieren und gewünschtenfalls in ein Salz überführen kann.

4. Arzneimittel, dadurch gekennzeichnet, daß sie aus dem Produkt der Formel (I), wie in Anspruch 1 definiert, sowie aus dessen Additionssalzen mit pharmazeutisch verträglichen Säuren bestehen.

5. Pharmazeutische Zusammensetzungen, dadurch gekennzeichnet, daß sie als Wirkstoff das Produkt der Formel (I), wie in Anspruch 1 definiert, oder eines seiner Additionssalze mit pharmazeutisch verträglichen Säuren enthalten.

6. Verwendung des Produkts der Formel (I), wie in Anspruch 1 definiert, als Mittel zur Untersuchung, Bestimmung und Lokalisierung von opiumhaltigen Kapparezeptoren.

7. Verwendung des Produkts der Formel (I), wie in Anspruch 1 definiert, und seiner Additonssalze mit pharmazeutisch verträglichen Säuren für die Herstellung von pharmazeutischen Zusammensetzungen zur Behandlung der Überdosierung eines agonistischen Produkts des opiumhaltigen Kapparezeptors.

8. Als neues industrielles Produkt das Produkt der Formel (II), wie in Anspruch 3 definiert.

**Patentansprüche für folgenden Vertragsstaat : ES**

1. Verfahren zur Herstellung des Derivats der Formel (I)

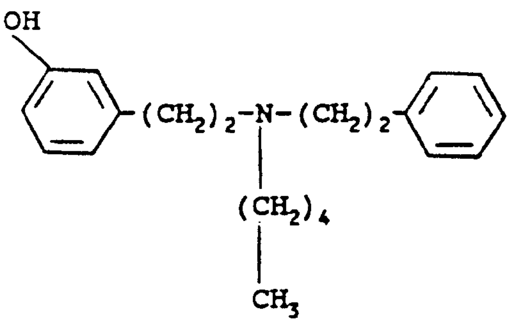

(I)

sowie von dessen Additionssalzen mit Mineral- oder organischen Säuren, dadurch gekennzeichnet, daß man das Produkt der Formel (II)

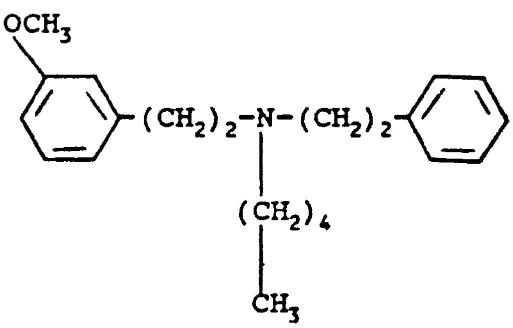

(II)

entmethyliert, um zu dem Produkt der Formel (I) zu gelangen, das man in Form der Base isolieren und gewünschtenfalls in ein Salz überführen kann.

2. Verfahren gemäß Anspruch 1, dadurch gekennzeichnet, daß man die Base mit Chlorwasserstoffsäure in ein Salz überführt.

3. Verwendung des Produkts der Formel (I), wie in Anspruch 1 definiert, und von seinen Additionssalzen mit pharmazeutisch verträglichen Säuren für die Herstellung von pharmazeutischen Zusammensetzungen zur Behandlung der Überdosierung eines agonistischen Produkts des opiumhaltigen Kapparezeptors.

**Patentansprüche für folgenden Vertragsstaat : GR**

1. Verfahren zur Herstellung des Derivats der Formel (I)

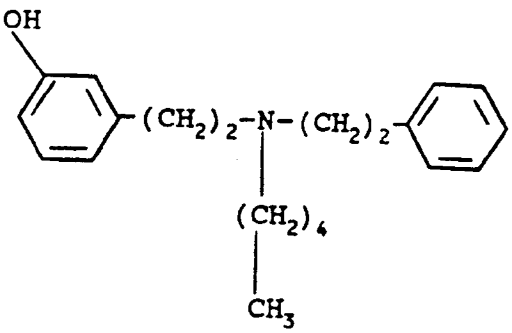

(I)

sowie von dessen Additionssalzen mit Mineral- oder organischen Säuren, dadurch gekennzeichnet, daß man das Produkt der Formel (II)

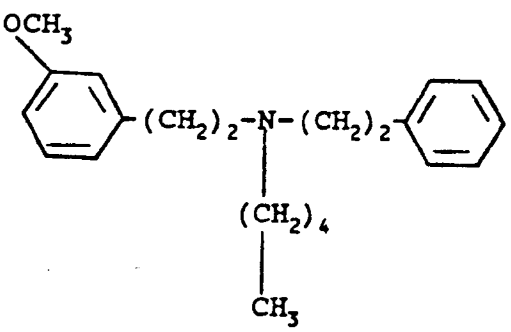

(II)

entmethyliert, um zu dem Produkt der Formel (I) zu gelangen, das man in Form der Base isolieren und gewünschtenfalls in ein Salz überführen kann.

**2.** Verfahren gemäß Anspruch 1, dadurch gekennzeichnet, daß man die Base mit Chlorwasserstoffsäure in ein Salz überführt.

**3.** Als neues industrielles Produkt das Produkt der Formel (II), wie in Anspruch 1 definiert.

**4.** Verfahren zur Herstellung von pharmazeutischen Zusammensetzungen, dadurch gekennzeichnet, daß man als Wirkstoff das Produkt der Formel (I) oder eines seiner Additionssalze mit pharmazeutisch verträglichen Mineral- oder organischen Säuren in eine für diese Verwendung bestimmte Form bringt.

**5.** Verwendung des Produkts der Formel (I), wie in Anspruch 1 definiert, und von seinen Additionssalzen mit pharmazeutisch verträglichen Säuren für die Herstellung von pharmazeutischen Zusammensetzungen zur Behandlung der Überdosierung eines agonistischen Produkts des opiumhaltigen Kapparezeptors.

**Claims**

**Claims for the following Contracting States : AT, BE, CH, DE, DK, GB, IT, LI, LU, NL, SE**

**1.** The new derivative of hydroxyphenethylamine characterized in that it corresponds to formula (I):

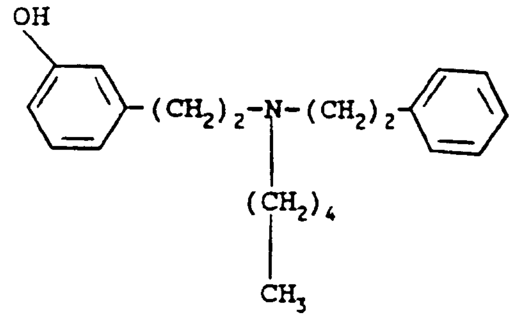

(I)

as well as its addition salts with the mineral or organic acids.

2. - N-pentyl N-phenethyl 3-hydroxy phenethylamine
   - N-pentyl N-phenethyl 3-hydroxy phenethylamine hydrochloride.

3. Preparation process for the product of formula (I) as defined in claim 1, as well as its addition salts with mineral or organic acids, characterized in that the product of formula (II):

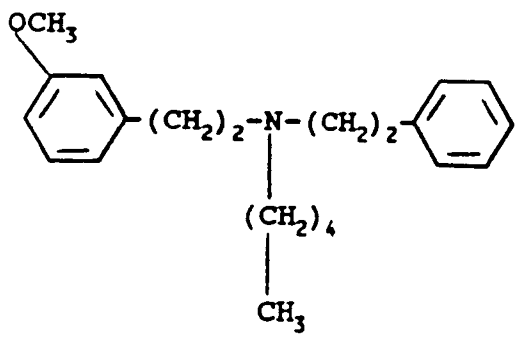

(II)

is demethylated in order to obtain the product of formula (I) which can be isolated in the form of the base and, if desired, salified.

4. Medicaments, characterized in that they are constituted by the product of formula (I) as defined in claim 1 as well as by its addition salts with pharmaceutically acceptable acids.

5. Pharmaceutical compositions characterized in that they contain, as active ingredient, the product of formula (I) as defined in claim 1 or one of its addition salts with pharmaceutically acceptable acids.

6. Use of the product of formula (I) as defined in claim 1 as a means of studying, dosing and localization of kappa opiate receptors.

7. Use of the product of formula (I) as defined in claim 1 and its addition salts with pharmaceutically acceptable acids for the preparation of pharmaceutical compositions intended for the treatment of an overdose with an agonist product of the kappa opiate receptor.

8. As a new industrial product, the product of formula (II) as defined in claim 3.

**Claims for the following Contracting State : ES**

1. Process for the preparation of the derivative corresponding to formula (I):

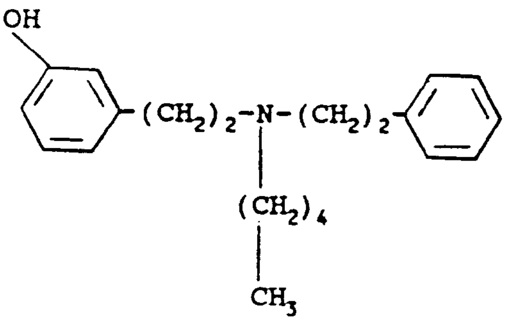

(I)

as well as its addition salts with mineral or organic acids, characterized in that the product of formula (II):

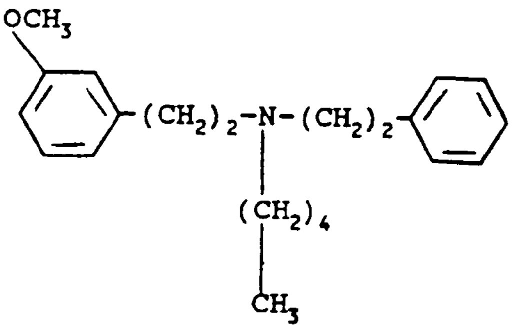

(II)

is demethylated in order to obtain the product of formula (I) which can be isolated in the form of the base and, if desired, salified.

2. Process according to claim 1, characterized in that the base is salified with hydrochloric acid.

3. Use of the product of formula (I) as defined in claim 1 and its addition salts with pharmaceutically acceptable acids for the preparation of pharmaceutical compositions intended for the treatment of an overdose with an agonist product of the kappa opiate receptor.

**Claims for the following Contracting State : GR**

1. Process for the preparation of the derivative corresponding to formula (I):

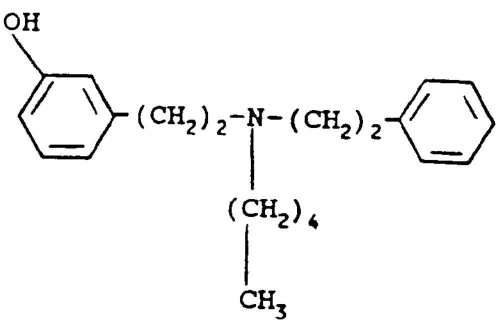

(I)

as well as its addition salts with mineral or organic acids, characterized in that the product of formula (II):

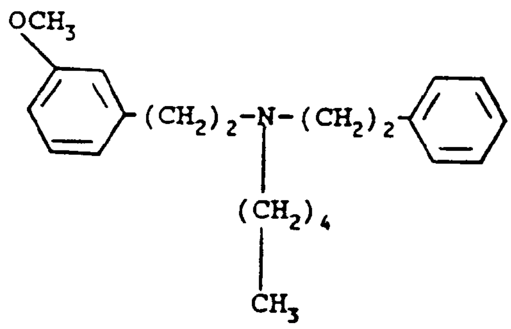

(II)

is demethylated in order to obtain the product of formula (I) which can be isolated in the form of the base and, if desired, salified.

**2.** Process according to claim 1, characterized in that the base is salified with hydrochloric acid.

**3.** As a new industrial product, the product of formula (II) as defined in claim 1.

**4.** Preparation process for pharmaceutical compositions characterized in that the product of formula (I) or one of its addition salts with pharmaceutically acceptable mineral or organic acids is used as active ingredient, in a form intended for this use.

**5.** Use of the product of formula (I) as defined in claim 1 and its addition salts with pharmaceutically acceptable acids for the preparation of pharmaceutical compositions intended for the treatment of an overdose with an agonist product of the kappa opiate receptor.